# EUROPÄISCHE PATENTSCHRIFT

(11) **EP 1 133 954 B1**
(45) Veröffentlichungstag und Bekanntmachung des Hinweises auf die Patenterteilung: **25.05.2005**
(21) Anmeldenummer: 01103658.9
(22) Anmeldetag: 23.02.2001
(51) Int. Cl.: A61C 1/00

(54) **Dentalgerät**
Dental apparatus
Appareil dentaire

(30) Priorität: 15.03.2000 DE 10012632
(43) Veröffentlichungstag der Anmeldung: 19.09.2001
(73) Patentinhaber: Ivoclar Vivadent AG, 9494 Schaan (LI)
(72) Erfinder: Grünenfelder, Robert, 9492 Eschen (LI); Lorünser, Johannes, 6700 Bludenz (AT); Rohner, Gottfried, 9450 Alstätten (CH)
(74) Vertreter: Baronetzky, Klaus, Dipl.-Ing.

(56) Entgegenhaltungen:
- EP-A- 0 503 890
- US-A- 5 788 485

## Beschreibung

Die Erfindung betrifft ein Dentalgerät für das Durchführen eines Dentalprozesses, gemäß dem Oberbegriff von Anspruch 1.

Derartige Dentalgeräte werden in weiten Bereichen eingesetzt, wenn es gilt, Dentalprozesse rationell durchzuführen. Ein Beispiel hierfür ist ein Ausbrühgerät für das bei der Herstellung einer Prothese verwendete Wachs, oder ein Dampfstrahlgerät für das Reinigen der Prothese. Ein Ausbrühgerät erwärmt ein Modell über den Schmelzpunkt des Wachses hinaus, jedoch eindeutig unterhalb des Schmelzpunktes sonstiger Materialien, die für die Herstellung der vorliegenden Prothese verwendet werden.

Zwar erfolgt nach Abschluß des Ausbrühvorgangs ein Abkühlen. Ohne Eingriff einer Bedienperson verbleibt das Modell aber zunächst noch relativ warm in dem Ausbrühgerät, wodurch es einer gewissen Belastung, wenn nicht sogar Schädigung, unterworfen wird. Daher wäre es günstig, wenn die Bedienperson den Ausbrühvorgang abwartet, um zeitgerecht das Modell entnehmen zu können. Andererseits ist es wünschenswert, daß qualifizierte Zahntechniker ihre Arbeitszeit nicht mit Warten verbringen, und regelmäßig könnte der Zahntechniker auch andere Arbeiten vornehmen, wenn er nicht das Dentalgerät überwachen müßte.

Eine ähnliche Situation ergibt sich auch bei vielen weiteren Geräten des Dentalbereichs, beispielsweise Sterilisiergeräten, Vorwärmöfen, Dampfstrahlgeräten, aber auch bei Galvanisiergeräten, Gießgeräten oder Fräsgeräten sowie Polymerisationsgeräten.

Darüber hinaus werden auch Öfen wie z.B. Pressöfen als Dentalgeräte heute in großem Umfang eingesetzt, um keramische Restaurationen wie z.B. Kronen oder Brücken in einer sogenannten Muffel zu brennen. Die zu sinternde Keramik wird in der Muffel in den Ofen eingebracht und dort einem exakt definierten Brennvorgang mit genauen Werten hinsichtlich des aufgebrachten Drucks und hinsichtlich der aufgebrachten Temperatur unterworfen. Die Technik der Herstellung von Prothesenteilen ist heutzutage recht verfeinert, denn es gilt, eine hohe Maßhaltigkeit zu gewährleisten, um also eine entsprechend gute Paßform zu erzielen. Auch wenn die Abformung, die Modellierung, die Erstellung des Negativabdrucks und die weiteren erforderlichen Schritte für die Herstellung der Gießform realisiert werden müssen, stellt der Pressofen häufig einen gewissen Engpaß bei der Prothesenfertigung dar, zumal diese Brennöfen aufgrund der erforderlichen aufwendigen Technik, der besonderen Steuerung, der Druckabdichtung usw. zu den eher werthaltigen Einrichtungen in Dentallabors zählen, die nicht in beliebiger Menge angeschafft werden.

Um den Durchsatz zu verbessern, aber auch aus anderen Gründen, stellt man häufig sog. Vorwärmöfen beim Pressen von Keramik bereit, in denen die Muffel auf eine bestimmte Vorwärmtemperatur, beispielsweise 700 °C, aufgewärmt wird. Die Muffel kann dann in bereits warmem Zustand in den Pressofen eingebracht werden, so daß die Dauer des Brennzyklus entsprechend verringert wird.

Dentallabors umfassen in der Regel mehrere Räume, wobei funktionell zusammengehörende Geräte, wie der Pressofen und der Vorwärmofen, regelmäßig benachbart angeordnet sind. Der Zahntechniker hält sich dann in diesem - meist eher warmen - Raum auf, solange er mit der Bedienung dieser Geräte oder weiterer zugeordneter, im gleichen Raum befindlicher Geräte beschäftigt ist und verläßt dann den Raum.

Damit der Zahntechniker den Brennofen nicht stets im Auge behalten muß, öffnet sich der Brennofen beim Ende des Brennzyklus' automatisch, so daß bereits die Abkühlung der Muffel beginnt. Zusätzlich beginnt aber auch der Ofen selbst auszukühlen. Den entsprechenden Wärmeverlust und damit die erheblichen Verluste für das Wiederaufwärmen, die auch zu entsprechender Zeitverzögerung führen, müssen in Kauf genommen werden, denn es ist wichtig, die Muffel nicht zu überbrennen, also die Muffel nach dem Ende des Brennvorgangs in dem geschlossenen und daher heißen Brennofen zu belassen.

Das Dokument US 5 788 485 offenbart ein Dentalgerät nach dem Oberbegriff von Anspruch 1.

Daher ist es vorgeschlagen worden, Brennöfen mit akustischen Signalen zu versehen, die das Ende des Brennvorgangs anzeigen sollen. Ein derartiges akustisches Signal wirkt allerdings nur dann zuverlässig, wenn eine entsprechende Lautstärke bereitgestellt wird. Wenn ein Zahntechniker in einem anderen Raum an einem Gerät, z.B. einem Mischer, tätig ist, das entsprechend starke Eigengeräusche von sich gibt, vermag er das akustische Signal nur dann wahrzunehmen, wenn es entsprechend laut ist, so daß es auch in den Raum herüberdringt, in dem der Zahntechniker gerade tätig ist. Ein derart lautes Signal wirkt aber dann ausgesprochen störend, wenn der Zahntechniker sich ohnehin in dem Raum des Pressofens befindet.

Ferner ist es im Bereich der Gießereitechnik, nämlich bei Schmelzöfen für Aluminium u.a., vorgeschlagen worden, spezielle Alarmanlagen einzusetzen, die eine Fehlfunktion des Ofens und insbesondere ein zu frühes erneutes Einschalten signalisieren und ggf. verhindern sollen. Nachdem eine Aluminiumhütte sich über eine beträchtliche Ausdehnung erstrecken kann, ist es in diesem Zusammenhang auch vorgeschlagen worden, die Signalisierung des Alarms entfernt vorzusehen, beispielsweise über eine Telephonverbindung, eine feste Funkstrecke oder über eine Standleitung. Allerdings liegen hier insofern keine vergleichbaren Verhältnisse vor.

Nachteilig bei den bekannten Brennöfen für Dentalmaterial ist es ferner, daß der Zahntechniker, der während des Brennvorgangs sich anderen Arbeiten zuwendet, diese unter der gewissen Anspannung vornehmen muß, um nicht das Ende des Brennvorgangs zu verpassen bzw. ggf. ein akustisches Signal zu überhören. Zwar könnte der Zahntechniker einen Kurzzeitwecker als Hilfsmittel für die Zeitabschätzung verwenden. Nachdem ein derartiger Wecker ansonsten im Dentallabor nicht benötigt wird, müßte er gesondert beschafft werden. Wichtiger in diesem Zusammenhang ist es allerdings, daß bei der Einstellung und dem erforderlichen Zeitabgleich zwischen Wecker und Brennbeginn eine Unsicherheit eingebracht wird, zumal die unterschiedlichen Brennprogramme unterschiedliche Laufzeiten haben, die teilweise auch von der Ausgangstemperatur des Brennofens abhängen, die der Zahntechniker dementsprechend nicht ganz genau abschätzen kann. Ein Kurzzeitwecker ist als Hilfmittel in diesem Zusammenhang insofern eher untauglich.

Daher liegt der Erfindung die Aufgabe zugrunde, ein Dentalgerät für das Durchführen eines Dentalprozesses gemäß dem Oberbegriff von Anspruch 1 zu schaffen, das eine schnellere Bedienbarkeit für die rationelle Durchführung von weiteren Tätigkeiten im Dentallabor ermöglicht. Die Wiederbenutzung des Dentalgeräts soll schneller als bisher ermöglicht werden. Die rechtzeitige Entnahme des Behandlungsgutes soll sicherer werden.

Diese Aufgabe wird erfindungsgemäß durch Anspruch 1 gelöst. Vorteilhafte Weiterbildungen ergeben sich aus den Unteransprüchen.

Erfindungsgemäß besonders günstig ist es, daß das erfindungsgemäße Dentalgerät zeitlich optimal ausgenutzt werden kann, ohne die Produktivität des Zahntechnikers oder der sonstigen Bedienperson zu beeinträchtigen. Daduch läßt sich verhindern, daß das Dentalobjekt auch nach dem Abschalten des Dentalgeräts in diesem verbleibt und ggf. etwas in Mitleidenschaft gezogen wird.

Mit der erfindungsgemäßen Lösung läßt sich sicherstellen, daß der Zahntechniker stets zum richtigen Zeitpunkt auf das Prozeßende des Dentalgeräts aufmerksam gemacht wird, so daß er an seinem Arbeitsplatz verbleiben und ungestört arbeiten kann, ohne auf den Brennvorgang achtgeben zu müssen. Erfindungsgemäß ist hierzu eine drahtlose Personenrufvorrichtung eingesetzt, die einen Sender umfaßt, der mit der Programmsteuerung des Dentalgeräts verbunden ist und bei Prozeßende oder in zeitlicher Nähe zum Ende des Prozesses aktiviert wird.

Der Sender weist eine bestimmte Kodierung auf, auf die der Empfänger anspricht. Der Empfänger wird von dem Zahntechniker mitgeführt und kann ausgesprochen klein sein, beispielsweise nur wenige cm lang. Er signalisiert das Prozeßende akustisch, optisch und/oder durch Vibration. Überraschend läßt sich mit dieser einfachen Maßnahme gewährleisten, daß die Benutzung des Dentalgeräts, aber auch der Energieverbrauch optimiert wird. Es ist ausgeschlossen, daß z.B. ein Ofen minutenlang offen verbleibt und damit auskühlt, ohne daß der Zahntechniker es bemerken würde.

Gemäß einem erfindungsgemäß günstigen zusätzlichen Gesichtspunkt läßt sich mit dem erfindungsgemäßen Dentalgerät mit drahtloser Personenrufvorrichtung eine eindeutige Identifizierung des rufenden Dentalgeräts sicherstellen. Hierzu wird die Kodierung eingesetzt. Wenn beispielsweise ein weiteres Dentalgerät in räumlicher Nähe zu dem betrachteten Dentalgerät installiert ist, für den der Zahntechniker aber nicht, sondern ein anderer Zahntechniker zuständig ist, kann dies von der Kodierung her ohne weiteres berücksichtigt werden, so daß der Empfänger des Zahntechnikers je nur auf den Sender des Dentalgeräts anspricht, für den er zuständig ist.

Gemäß einer vorteilhaften Ausgestaltung ist es auch möglich, mehrere Dentalgeräte parallel dahingehend zu überwachen, daß jedes Dentalgerät an den gleichen Empfänger, der für zwei unterschiedliche Codes empfangbereit ist, unterschiedliche Signalisierungen auslöst, um die Dentalgeräte, deren Prozeßende erreicht ist, unterscheidbar zu machen.

Ferner ist es auch möglich, über weitere unterschiedliche Signalisierungen den Prozeßstatus zu übergeben. So kann die Bedienungsperson darüber informiert gehalten werden, ob der Prozeß in störungsfreier Form abläuft, oder ob ein Fehler entstanden ist.

Weitere Vorteile, Einzelheiten und Merkmale ergeben sich aus der nachfolgenden Beschreibung eines Ausführungsbeispiels der Erfindung anhand der Zeichnung. Als Beispiel für ein Dentalgerät wird ein Preßofen für Keramik beschrieben. Es zeigen:
- Fig. 1: eine schematische Ansicht eines Ofens als Beispiel eines erfindungsgemäßen Dentalgeräts, an dem ein Sender einer erfindungsgemäßen Personenrufvorrichtung angeflanscht ist;
- Fig. 2: eine schematische Darstellung eines Empfängers der Personenrufvorrichtung; und
- Fig. 3: das Prinzipschaltbild des erfindungsgemäßen Ofens mit der Personenrufvorrichtung.

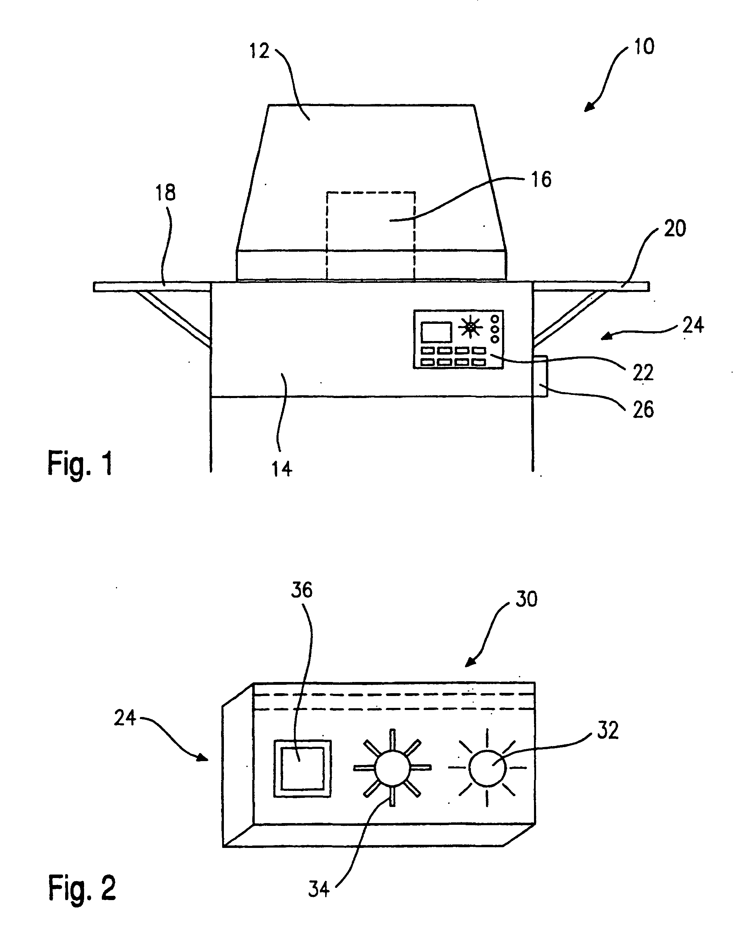

Der in Fig. 1 dargestellte Ofen 10 weist eine Brennhaube 12 und ein Unterteil 14 auf. Eine gestrichelt dargestellte Muffel 16 steht auf einer Brenngutplatte des Unterteils 14 und ist von der Brennhaube 12 umgeben. Die Brennhaube 12 weist innen in an sich bekannter Weise eine Heizvorrichtung auf, die die Muffel allseitig umgibt und erwärmt.

Der in Fig. 1 dargestellte Ofen 10 weist zwei Arbeitsflächen 18 und 20 auf, die dazu dienen, die nächste Muffel bereitzustellen und nach Brennende die fertige Muffel herauszuschieben. Hierzu wird die Brennhaube 12 angehoben, so daß sie die Muffel 16 freigibt. Dies geschieht automatisch über eine Programmsteuerung, die in den Ofen 10 untergebracht ist. Die Programmsteuerung wird über ein Bedienfeld 22 bedient, das an der Vorderseite des Ofens angebracht ist und auch verschiedene Anzeigen erlaubt, beispielsweise auch ein graphisches Anzeigefeld für die gewünschten Brennkurven, die programmiert werden sollen.

Beim Ende des Brennvorgangs öffnet sich die Brennhaube 12 automatisch, wobei sie zunächst leicht angehoben und dann nach oben geschwenkt wird. In diesem Zustand kühlt die Muffel 16 bereits aus, und es wäre möglich, sie zu entfernen und auf der Arbeitsfläche 20 auskühlen zu lassen und die nächste Muffel von der Arbeitsfläche 18 einzuschieben.

Erfindungsgemäß ist eine Personenrufvorrichtung 24 vorgesehen, deren Sender 26 in Fig. 1 schematisch dargestellt ist. Der Sender 26 ist mit der Programmsteuerung verbunden und wird aktiviert, kurz bevor der Brennofen 10 den Brennvorgang abgeschlossen hat und die Brennhaube 12 öffnet. Es versteht sich, daß der zeitliche Abstand zum tatsächlichen Ende des Brennvorgangs in weiten Bereichen an die Erfordernisse angepaßt werden kann. Beispielsweise bietet sich ein Zeitraum von minus 5 Sekunden an, also 5 Sekunden, bevor sich die Brennhaube 12 öffnet. Bei dem Preßofen wird das Ende des Prozesses direkt angezeigt.

In einer weiteren Ausgestaltung der Erfindung ist es vorgesehen, den Zeitraum wählbar zu halten, so daß der Benutzer selbst einstellen kann, wieviel Vorwarnzeit er haben möchte, oder ob überhaupt eine Vorwarnzeit erwünscht ist.

Der Sender gibt ein kodiertes Signal ab. Hierzu kann das Trägersignal amplitudenmoduliert, frequenzmoduliert, oder beispielsweise pulsweitenmoduliert werden, wobei die eingestellte Kodierung bei Bedarf auch veränderlich ausgebildet sein kann, um beispielsweise den Parallelbetrieb mehrerer Öfen zu ermöglichen.

In Fig. 2 ist ein Empfänger 30 der Personenrufvorrichtung 24 dargestellt. Der Empfänger 30 ist eine ausgesprochen schlanke Einheit von beispielsweise 2 cm Länge, 1 cm Höhe und ist beispielsweise 5 mm flach. Er weist einen Dekodierer auf, der auf den Kodierer im Sender 26 abgestimmt ist, so daß er aktiviert wird, wenn der Sender das für den Empfänger 30 bestimmte Signal abgibt. In dem dargestellten Ausführungsbeispiel erfolgt die Signalisierung sowohl optisch über eine Leuchtdiode 32 als auch akustisch über einen Summer 34. Ferner ist ein kleiner Knopf 36 vorgesehen, der auf einen Taster wirkt, mit dem die Signale des Summers und der Leuchtdiode abschaltbar sind. Der Empfänger 30 ist batteriebetrieben und weist trotz seiner Kompaktheit auch eine kleine Antenne auf, um die übermittelte Information über den erforderlichen Abstand erfassen zu können.

Sobald der Zahntechniker ein Signal des Empfängers wahrnimmt, schaltet er das Signal aus und begibt sich zu dem rufenden Ofen, um die Muffel 16 zu entnehmen und ggf. die nächste Muffel einzusetzen. Hieran anschließend kann er umgehend die unterbrochene Arbeit fortsetzen. Dies gilt gleichermaßen, wenn er sich bereits in dem Ofenraum befindet.

In einer modifizierten Ausgestaltung des Empfängers ist es vorgesehen, daß ein Vibrationselement eingebaut ist, das dann seine Signalfunktion auszuüben vermag, wenn der Empfänger beispielsweise in der Hemdtasche getragen wird.

In einer weiteren modifizierten Ausgestaltung ist es vorgesehen, durch mehrfache Betätigung des Knopfes 36 die Art der Signalisierung zyklisch sequentiell umzuschalten, also etwa akustisch - optisch - akustisch+optisch.

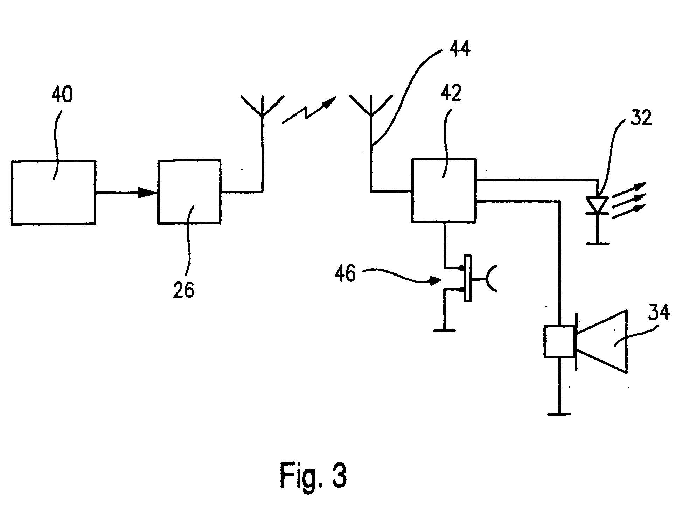

In Fig. 3 ist das Prinzipschaltbild einer erfindungsgemäßen Personenrufvorrichtung dargestellt. Eine Programmsteuerung 40 steuert den Sender 26 an, der seinerseits mit einer Sendeantenne verbunden ist. Wenn eine Vorlaufzeit zwischen Signalisierung und Brennende gewünscht ist, empfiehlt es sich, das Steuersignal für den Sender nicht unmittelbar aus dem Programmende zu gewinnen, sondern aus der Programmsteuerung selbst, die ohnehin über eine entsprechende Steuerlogik und Zeitmessung verfügt.

Der Empfänger 30 weist einen Empfangsschaltkreis 42 auf, der mit einer Empfangsantenne 44 verbunden ist. Sein Ausgang ist sowohl mit der Leuchtdiode 32 als auch mit dem Summer 34 verbunden. Es versteht sich, daß anstelle des Summers 34 auch ein kleiner Lautsprecher eingesetzt werden kann. Der Empfangsschaltkreis 42 weist auch die Dekodiervorrichtung auf. Ferner ist er mit einem Taster 46 verbunden, der über den Knopf 36 betätigt wird. Beim Betätigen des Knopfes 36 wird die Anzeige für eine gewisse Zeit, beispielsweise 10 Sekunden, unterbunden, so daß der Zahntechniker genug Zeit hat, die erforderlichen Schritte am Ofen vorzunehmen. Das Sendesignal wird erfindungsgemäß durch eine beliebige Bedienfunktion, die der Zahntechniker an dem Ofen ausübt, unterdrückt. Wenn er beispielsweise den Ofen abschaltet, weil kein weiterer Brennvorgang erforderlich ist, wird auch das Signal für die Sendevorrichtung abgeschaltet. Dies gilt gleichermaßen für das Starten eines neuen Brennzyklus' mit der nächsten Muffel. In beiden Fällen ist es erfindungsgemäß günstig, wenn der Empfänger erst dann wieder aktiviert wird, also "scharf" ist, wenn die übliche Bedienzeit für den entsprechenden Vorgang überschritten ist, so daß insofern kein Fehlalarm entsteht.

Es versteht sich, daß weitere Modifikationen möglich sind, ohne den Bereich der Erfindung zu verlassen. So kann die erfindungsgemäße Lösung ohne weiteres auch an beliebigen anderen dentalen Geräten außer Brenn- und Pressöfen eingesetzt werden, bei denen eine rationelle Fertigung angestrebt wird und bei denen die betreffende Zahnrestauration für einen längeren Zeitraum in dem betreffenden Gerät verbleibt und automatisch fertiggestellt wird.

Gemäß einer weiteren, besonders günstigen Ausgestaltung der Erfindung ist es vorgesehen, das Ende des Prozesses über eine vorhandene Verbindungsresource signalisieren zu lassen. Beispielsweise kann das Nachrichtenübermittlungssystem eines Netzwerks von vorhandenen PCs hierzu eingesetzt werden. Eine andere Möglichkeit ist die Signalisierung über besondere Leitungen, über welche die Dentalgeräte Daten miteinander austauschen können.

## Patentansprüche

1. Dentalgerät für das Durchführen eines Dental-Prozesses, mit einer Programmsteuerung, die mindestens eine Parameterkurve des Dentalgeräts während des Prozesses steuert, mit einer Abschaltvorrichtung, die das Dentalgerät in zeitlichem Abstand vom Beginn des Prozesses beim Ende des Prozesses abschaltet, **dadurch gekennzeichnet, daß** an die Programmsteuerung (40) eine drahtlose Personenrufvorrichtung (24) angeschlossen ist, die in zeitlicher Nähe zum Ende des Prozesses oder direkt bei Prozeßende aktivierbar ist.

2. Dentalgerät nach Anspruch 1, **dadurch gekennzeichnet, daß** die drahtlose Personenrufvorrichtung (24) kurz vor dem Ende des Prozesses aktivierbar ist.

3. Dentalgerät nach einem der vorhergehenden Ansprüche, **dadurch gekennzeichnet, daß** die drahtlose Personenrufvorrichtung (24) einen Sender (26) aufweist, der mit einem Schaltausgang der Programmsteuerung (40) verbunden ist.

4. Dentalgerät nach einem der vorhergehendem Ansprüche, **dadurch gekennzeichnet, daß** die drahtlose Personenrufvorrichtung (24) einen mobilen, batterie- oder akkubetriebenen Empfänger (30) mit einer akustischen und/oder optischen Anzeige oder eine Klopfanzeige aufweist.

5. Dentalgerät nach einem der vorhergehenden Ansprüche, **dadurch gekennzeichnet, daß** der Sender (26) der drahtlosen Personenrufvorrichtung eine Kodiervorrichtung und der Empfänger (30) eine Dekodiervorrichtung aufweist, und daß der Empfänger (30) eine eindeutige Identifizierung des signalisierenden Dentalgeräts (10) erlaubt.

6. Dentalgerät nach einem der vorhergehenden Ansprüche, **dadurch gekennzeichnet, daß** die Personenrufvorrichtung (24) auch einen etwaigen Dentalgerätsfehler signalisiert, insbesondere mit einem von dem Signal für das Ende des Prozesses unterschiedlichen Signal.

7. Dentalgerät nach einem der vorhergehenden Ansprüche, **dadurch gekennzeichnet, daß** die drahtlose Personenrufvorrichtung (24) einen modularen Sender (26) aufweist, mit dem bestehende Dentalgeräte nachrüstbar sind.

8. Dentalgerät nach einem der vorhergehenden Ansprüche, **dadurch gekennzeichnet, daß** der Empfänger (30) eine Löschtaste aufweist, mit welcher der Alarm nach Kenntnisnahme durch den Benutzer löschbar ist.

9. Dentalgerät nach einem der vorhergehenden Ansprüche, **dadurch gekennzeichnet, daß** die Sendeleistung des Senders (26) weniger als 500 Milliwatt, insbesondere etwa 50 Milliwatt beträgt, und die Reichweite auf etwa 100 m beschränkt ist.

10. Dentalgerät nach einem der vorhergehenden Ansprüche, **dadurch gekennzeichnet, daß** das Dentalgerät als Brenn- oder Pressofen für eine Dentalkeramik, oder als Vorwärmofen für die Dentalkeramik ausgebildet ist.

11. Dentalgerät nach einem der Ansprüche 1 bis 9, **dadurch gekennzeichnet, daß** das Dentalgerät als Polymerisationsgerät zum Aushärten von Zahnrestaurationen oder als Ausbrüh- oder Dampfstrahlgerät für die Herstellung von Dentalprothesen ausgebildet ist.

12. Dentalgerät nach einem der Ansprüche 1 bis 9, **dadurch gekennzeichnet, daß** das Dentalgerät als Fräsgerät, insbesondere in CAD/CAM-Technik, oder als Galvanisiergerät zur Herstellung von Dentalrestaurationen ausgebildet ist.

13. Dentalgerät nach einem der Ansprüche 1 bis 9, **dadurch gekennzeichnet, daß** das Dentalgerät als Dosier-, Misch-, Gießoder Sterilisiergerät für die Herstellung von Zähnen und/oder Zahnrestaurationen ausgebildet ist.

14. Dentalgerät nach einem der vorhergehenden Ansprüche, **dadurch gekennzeichnet, daß** die Zeit zwischen Prozeßanfang und Prozeßende zwischen 10 Minuten und 10 Stunden, insbesondere zwischen 15 Minuten und 2 Stunden beträgt.

15. Dentalgerät nach einem der vorhergehenden Ansprüche, **dadurch gekennzeichnet, daß** die Personenrufvorrichtung (24) als Nachrichtenübermittlungsvorrichtung ausgebildet ist, die über eine Verbindungsresource wie beispielsweise ein hausinternes Netzwerk und dessen Nachrichtenübermittlungsfunktion das Ende des Prozesses signalisiert.

## Claims

1. A dental apparatus for carrying out a dental process, having a program control which controls at least one parameter curve of the dental apparatus during the process, with a switch-off device which switches off the dental apparatus at the end of the process at a time interval from the start of the process, **characterised in that** a wireless paging device (24) is connected to the program control (40), which can be activated in time proximity to the end of the process or directly at the end of the process.

2. A dental apparatus according to Claim 1, **characterised in that** the wireless paging device (24) can be activated just before the end of the process.

3. A dental apparatus according to any one of the preceding Claims, **characterised in that** the wireless paging device (24) has a transmitter (26) which is connected to a switched output of the program control (40).

4. A dental apparatus according to any one of the preceding Claims, **characterised in that** the wireless paging device (24) has a mobile, battery-powered receiver (30) with an acoustic indicator and/or visual display or a sounder.

5. A dental apparatus according to any one of the preceding claims, **characterised in that** the transmitter (26) of the wireless paging device has a coding device and the receiver (30) has a decoding device, and **in that** the receiver (30) makes possible clear identification of the signalling dental apparatus (10).

6. A dental apparatus according to any one of the preceding claims, **characterised in that** the paging device (24) also signals any faults in the dental apparatus, in particular with a signal differing from the signal for the end of the process.

7. A dental apparatus according to any one of the preceding Claims, **characterised in that** the wireless paging device (24) has a modular transmitter (26) which can be retrofitted to existing dental apparatus.

8. A dental apparatus according to any one of the preceding Claims, **characterised in that** the receiver (30) has a cancel button with which the alarm can be cancelled after recognition by the operator.

9. A dental apparatus according to any one of the preceding Claims, **characterised in that** the transmitting power of the transmitter (26) is lower than 500 milliwatt, in particular approximately 50 milliwatt, and its operating range is limited to approximately 100 m.

10. A dental apparatus according to any one of the preceding Claims, **characterised in that** the dental apparatus is in the form of a firing or pressing kiln for a dental ceramic, or a preheating kiln for the dental ceramic.

11. A dental apparatus according to any one of Claims 1 to 9, **characterised in that** the dental apparatus is in the form of a polymerisation device for curing dental restorations or of a scalding or steam-jet device for the production of dental prostheses.

12. A dental apparatus according to any one of Claims 1 to 9, **characterised in that** the dental apparatus is in the form of a milling device, in particular using CAD/CAM technology, or an electroplating device for the production of dental restorations.

13. A dental apparatus according to any one of Claims 1 to 9. **characterised in that** the dental apparatus is in the form of a metering, mixing, casting or sterilising device for the production of teeth and/or dental restorations.

14. A dental apparatus according to any one of the preceding Claims, **characterised in that** the time between the start of the process and end of the process is between 10 minutes and 10 hours, in particular between 15 minutes and 2 hours.

15. A dental apparatus according to any one of the preceding Claims, **characterised in that** the paging device (24) is in the form of a message handling device which signals the end of the process via an interconnection resource, for example an internal network, and its message handling function.

## Revendications

1. Appareil de technique dentaire pour l'exécution d'un processus de technique dentaire, avec une commande à programme, qui commande au moins une courbe de paramètres de l'appareil de technique dentaire pendant le processus, un dispositif d'arrêt qui, au bout d'un certain intervalle de temps après le début du processus, arrête l'appareil de technique dentaire à la fin du processus, **caractérisé en ce qu'**un dispositif de téléavertissement (24), qui est activable à l'approche de la fin du processus ou directement à la fin du processus, est relié à la commande à programme (40).

2. Appareil de technique dentaire selon la revendication 1, **caractérisé en ce que** le dispositif de téléavertissement (24) est activable peu avant la fin du processus.

3. Appareil de technique dentaire selon l'une quelconque des revendications précédentes, **caractérisé en ce que** le dispositif de téléavertissement (24) présente un émetteur (26) qui est relié à une sortie de commutation de la commande à programme (40).

4. Appareil de technique dentaire selon l'une quelconque des revendications précédentes, **caractérisé en ce que** le dispositif de téléavertissement (24) présente un récepteur (30) mobile, à piles ou à accumulateurs, avec un avertisseur sonore et/ou optique ou un avertisseur à vibrations.

5. Appareil de technique dentaire selon l'une quelconque des revendications précédentes, **caractérisé en ce que** l'émetteur (26) du dispositif de téléavertissement présente un dispositif de codage et le récepteur (30), un dispositif de décodage, et **en ce que** le récepteur (30) permet une identification univoque de l'appareil de technique dentaire (10) émettant le signal.

6. Appareil de technique dentaire selon l'une quelconque des revendications précédentes, **caractérisé en ce que** le dispositif de téléavertissement (24) signale également une éventuelle erreur de l'appareil de technique dentaire, en particulier par un signal différent du signal signalant la fin du processus.

7. Appareil de technique dentaire selon l'une quelconque des revendications précédentes, **caractérisé en ce que** le dispositif de téléavertissement (24) présente un émetteur modulaire (26) qui peut venir compléter des appareils de technique dentaire existants.

8. Appareil de technique dentaire selon l'une quelconque des revendications précédentes, **caractérisé en ce que** le récepteur (30) présente une touche d'extinction permettant à l'utilisateur d'éteindre l'alarme après en avoir pris connaissance.

9. Appareil de technique dentaire selon l'une quelconque des revendications précédentes, **caractérisé en ce que** la puissance d'émission de l'émetteur (26) est inférieure à 500 milliwatt, en particulier d'environ 50 milliwatt, et **en ce que** la portée est limitée à environ 100 m.

10. Appareil de technique dentaire selon l'une quelconque des revendications précédentes, **caractérisé en ce que** l'appareil de technique dentaire est conçu en tant que four de cuisson ou de pressée pour une céramique dentaire, ou en tant que four de préchauffage pour la céramique dentaire.

11. Appareil de technique dentaire selon l'une quelconque des revendications 1 à 9, **caractérisé en ce que** l'appareil de technique dentaire est conçu en tant qu'appareil de polymérisation pour le durcissement de restaurations dentaires ou en tant qu'appareil d'ébouillantage ou à jet de vapeur pour la fabrication de prothèses dentaires.

12. Appareil de technique dentaire selon l'une quelconque des revendications 1 à 9, **caractérisé en ce que** l'appareil de technique dentaire est conçu en tant qu'appareil de fraisage, en particulier dans la technique CAO/FAO, ou en tant qu'appareil de galvanoplastie pour la fabrication de restaurations dentaires.

13. Appareil de technique dentaire selon l'une quelconque des revendications 1 à 9, **caractérisé en ce que** l'appareil de technique dentaire est conçu en tant qu'appareil de dosage, de mélange, de moulage ou de stérilisation pour la fabrication de dents et/ou de restaurations dentaires.

14. Appareil de technique dentaire selon l'une quelconque des revendications précédentes, **caractérisé en ce que** l'intervalle de temps entre le début du processus et la fin du processus est compris entre 10 minutes et 10 heures, en particulier entre 15 minutes et 2 heures.

15. Appareil de technique dentaire selon l'une quelconque des revendications précédentes, **caractérisé en ce que** le dispositif de téléavertissement (24) est conçu en tant que dispositif de transmission d'information qui signale la fin du processus par l'intermédiaire d'une ressource de connexion telle que, par exemple, un réseau interne et sa fonction de transmission d'information.
